Europäisches Patentamt

European Patent Office

Office européen des brevets

(11) Publication number: **0 490 583 B1**

## EUROPEAN PATENT SPECIFICATION

(45) Date of publication of patent specification: **01.03.95**　(51) Int. Cl.⁶: **A61K 7/06**, A61K 7/48

(21) Application number: **91311345.2**

(22) Date of filing: **05.12.91**

(54) Hair treatment composition.

(30) Priority: **06.12.90 EP 90313225**

(43) Date of publication of application:
**17.06.92 Bulletin 92/25**

(45) Publication of the grant of the patent:
**01.03.95 Bulletin 95/09**

(84) Designated Contracting States:
**AT BE CH DE DK ES FR GB GR IT LI NL SE**

(56) References cited:
EP-A- 0 250 300　　WO-A-86/06586
WO-A-88/03799　　DE-A- 2 423 637
DE-A- 3 443 985　　FR-A- 2 099 582
FR-A- 2 159 383　　FR-A- 2 202 698

**PATENT ABSTRACTS OF JAPAN vol. 11, no.
246 (C-439)(2693) 11 August 1987**

(73) Proprietor: **UNILEVER PLC
Unilever House
Blackfriars
London EC4P 4BO (GB)**
(84) Designated Contracting States:
**GB**

(73) Proprietor: **UNILEVER N.V.
Weena 455
NL-3013 AL Rotterdam (NL)**
(84) Designated Contracting States:
**BE CH DE DK ES FR GR IT LI NL SE AT**

(72) Inventor: **Griffiths, Llyr Glyndwr
8 Erw Fach
Bryn-y-Baal,
Mold,
Clwyd CH7 6YD,
Wales (GB)**

(74) Representative: **Bristow, Stephen Robert et al
Unilever PLC
Patent Division
Colworth House
Sharnbrook
Bedford MK44 1LO (GB)**

**Description**

The present invention relates to a hair treatment composition, in particular to a post-wash leave-on hair-treatment composition.

A problem with hair is that it often appears greasy. This greasiness is caused by the presence of sebum on the hair fibres. Sebum is produced in the sebaceous glands and is then excreted onto the scalp near the roots and tends to spread over the hair. Generally the washing of the hair renders the hair visibly clean, whereafter it takes a few days until newly formed sebum is secreted onto the hair and spread amongst the hair-fibres by various mechanisms such that the hair regreases.

It is an object of the invention to provide a hair-treatment composition which reduces the perception of greasiness of hair. A further object of the invention is to provide a hair treatment composition which is intended to be applied to the hair after washing and/or conditioning and which is intended not to be rinsed out, but to stay on the hair for one or more days until the hair is washed again.

Surprisingly it has now been found that a reduced greasiness of the hair can be observed if after the washing and/or conditioning of the hair a hair treatment composition is applied thereto, said composition comprising an astringent material in combination with a viscosity control material for the sebum.

Accordingly the present invention provides a hair treatment composition for reducing greasiness of hair, comprising an astringent material in combination with a viscosity control material for sebum.

JP-A-62053917 discloses a mixture of a condensed tannin with an anti-microbial agent (triclosan). It does not mention inorganic or organic astringents or mixtures thereof.

FR-A-2159383 discloses a dry composition and makes no mention of organic astringent in the final composition.

DE-A-2423637 discloses the use of methyl cellulose with inorganic astringent. It does not disclose use of an organic astringent.

WO-A-8803799 discloses an insect repellant containing zinc sulphate and alcohol. No thickener is used.

WO-A-8606586 discloses zinc chloride in admixture with other materials, among the other materials are listed anti-bacterial agents (such as triclosan) and methyl celluloses, propylene glycol is also generally disclosed.

DE-A-3443985 discloses a composition containing zinc sulphate and a carboxymethyl cellulose. No disclosure of mixtures of organic and inorganic astringent materials is made.

FR-A-2202698 has as example 22 a mixture of propylene glycol at 2 or 5% with zinc oxide and an antimicrobial agent. The propylene glycol is used at below the level of 10% needed for significant astringent properties.

FR-A-2099582 contains ethyl alcohol with anti microbial agents. There does not appear to be a disclosure of an inorganic astringent.

_The astringent material_

Compositions in accordance with the present invention comprise an astringent material. Astringency is a well-known principle in cosmetics and the selection of suitable astringency materials for use in hair treatment compositions in accordance with the present invention is within the ability of the skilled person. Preferably astringent materials are capable of drawing together or to contract organic tissues and/or astringent materials are capable of precipitating protein from an aqueous solution.

Preferred astringent materials are selected from the group of hydrolysable tannins, phenolic acids associated with tannins, phenols associated with tannins, flavonoid compounds, natural extracts providing astringency, organic astringents and inorganic astringents.

Examples of hydrolysable tannins are gallotannic acid, chebulinic acid, hamameli tannin, acer tannin and $\beta$-D-glucogallin.

Examples of phenolic acids associated with tannins are gallic acid, m-digallic acid, dehydrodigallic acid, valonic acid, chebulic acid, hexahydroxydiphenic acid, ellagic acid, caffeic acid, p-coumaric acid, ferulic acid, sinapic acid and chlorogenic acid.

Examples of phenols associated with tannins are pinosylvin, piceatannol, resveratrol, tropolone, thymohydroquinone and carvacrol.

Examples of flavonoid compounds are flavan, isoflavan, catechin, mollisacacidin, leacocyanidin, leucodelphinidin, pelargonidin, cyanidin, delphinidin, chalocone and aurone.

Examples of natural extracts providing astringency are witch hazel, vitamin B6, Vitamin D, Vitamin H, apple juice, cucumber, herbs, lemon extract and yarrow extract.

Examples of organic astringents are C(1-6) mono-, di- or trihydroxy alcohols such as ethanol, isopropyl alcohol, propylene glycol and glycerol.

Examples of inorganic astringents are aluminum salts such as potash alum $K_2SO_4.Al_2(SO_4)3.2H_2O$, aluminium sulphate, aluminium halides such as aluminium chloride, aluminium (sub) acetate, aluminium acetotartrate, aluminium citrate and aluminium methionate, zinc salts such as zinc sulphate, zinc halide such as zinc chloride and zinc iodide, zinc oxide, zinc permanganate, zinc fluorosilicate, zinc phenolsulphonate, zinc caprylate, zinc propionate and zinc acetate, iron (III) salts such as ferric chloride and ferric sulphate, copper salts such as copper sulphate and copper citrate, and silver salts such as silver nitrate, silver lactate and silver picrate.

Compositions of the invention comprise at least one inorganic astringent material, preferably an astringent zinc salt, more preferably zinc sulphate or zinc chloride.

Compositions of the invention comprise an organic astringent material, present at a level of from 10-30 % by weight of the composition.

The composition of the invention comprises an astringent material other than an organic astringent material, the level of this is from 0.01 to 10 % by weight of the composition, preferably 0.05 to 5 %, more preferred 0.1 to 2 %. Preferred astringent materials for this purpose are inorganic astringent materials, more preferably zinc salts, most preferably zinc sulphate and zinc chloride.

In an especially preferred embodiment of the invention a mixture of organic astringent materials and inorganic astringent materials is used. Most preferred is the combined use of ethanol and zinc sulphate or zinc chloride.

### The viscosity control material

Compositions in accordance with the invention comprise one or more materials for controlling the viscosity of the sebum. For the purpose of the present invention a viscosity control material for the sebum is any material which is capable of reducing the thinning of the sebum or which is capable of increasing the viscosity thereof, the comparison being made with sebum not treated with a composition of the invention.

Preferably viscosity control materials for use in compositions according to the invention are selected from anti-microbial agents and polymeric thickening materials.

It is believed that anti-microbial agents function as viscosity control agents because they reduce the microbial decomposition of sebum whereby free fatty acids of lower viscosity would be produced. The reduced formation of free fatty acids leads to a reduction in the thinning of the sebum.

Suitable anti-microbial agents may for example be selected from the group of halogenated salicylanilides, halogenated carbanilides, halogenated bis-phenols, alkylbenzoacrylates, quaternary ammonium compounds, thiuram sulfides, dithiocarbamates, pyridinone-N-oxides, halogenated diphenyl ethers, halogenated anilides of thiophene carboxylic acids and chlorohexidines. Examples of materials from these groups are described in US 4,022,880.

Especially preferred anti-microbial materials for use in compositions in accordance with the present invention are halogenated diphenyl ethers, more preferred halogenated hydroxydiphenyl ethers, most preferred 2′,4,4′ trichloro-2-hydroxydiphenyl ether (trivial name triclosan).

Preferably the level of anti-microbial materials, if any, is up to 5 %, more preferred 0.01 to 2 %, most preferred 0.02 to 0.5 % by weight of the composition.

Suitable polymeric thickening materials for the sebum can be selected from proteinaceous polymeric thickeners such as for example gelatin; esters of fatty acids and polyethylene glycols such as for example the di-ester of stearic acid and a polyethylene glycol of molecular weight between 2,000 and 10,000, for example about 6,000; gum materials such as for example xantham gum and agar; (C1-4)alkyl-substituted cellulose ethers, for example methyl, ethyl or isopropyl (MS 2-3, preferably 3) cellulose ethers with a molecular weight of 500,000 to 1,500,000, for example a hydroxypropylcellulose (MS = 3) of molecular weight of about 830,000; higher fatty acid esters of amylose, polysaccharides, depolymerised cellulose, dextran and fatty acid esters of polyglycerols. Especially preferred is the use of polymeric thickening materials which are soluble in sebum.

It is believed that polymeric thickeners for the sebum function as viscosity control materials, because they are believed to decrease the mobility of the sebum, thereby reducing the tendency of the sebum to migrate along the hair.

The level of the polymeric thickener material, if any, is preferably up to 10 %, more preferably from 0.1 to 5 %, most preferably 0.2 to 2 % by weight of the composition. Preferably the total level of viscosity control agent is from 0.01 to 10 %, more preferably 0.1 to 5 %, most preferably 0.2 to 2 %.

An especially preferred embodiment of the invention relates to the combined use of an anti-microbial material and a polymeric thickener.

Other ingredients

Compositions in accordance to the invention may comprise in addition to the astringent material and the viscosity control material further optional ingredients. However, due to the intended use of the hair-treatment compositions, whereby the composition is applied to the hair and not rinsed out but left on the hair for one or more days until the hair is washed again, it is preferred that compositions of the invention contain no or only low levels of ingredients which are less desirable to be left on the hair for these periods. In particular, the hair treatment compositions preferably contain less than 5 % by weight of surfactant materials, more preferably less than 2 % or less than 1 %, most preferably the compositions are substantially free of surfactants.

Usually compositions of the invention will comprise an aqueous base. Preferably the water level is from 20-99.98 % , more preferably 50-99 %, most preferably 75-95 % by weight of the composition.

Other suitable optional ingredients may for example be selected from the group of colorants, perfumes, antidandruff ingredients, styling ingredients etc. Preferably the total level of ingredients other than water, astringent(s) and viscosity control material(s) is less than 10 % by weight of the composition, more preferably less than 5 %, most preferably less than 2 % by weight of the composition.

Physical form

Compositions of the invention may have any physical form which renders them suitable for application to moist or dry hair. For example compositions of the invention may be pastes, gels, sprays, liquids etc. Preferably compositions of the invention are liquids, most preferably transparent liquids.

Preferably the viscosity of the products according to the invention is less than 1,500 mPa.s at 21 $s^{-1}$ and ambient temperature, more preferably from 0.05 to 100 mPa.s, most preferably from 0.1 to 20 mPa.s. The pH of the composition is preferably from 3-10, more preferably 3.5-8, most preferably 4-6.

Compositions of the invention generally will be packed in a container of 2-1,000 mls, more preferred 50-500 mls, for sale.

Use

Compositions of the invention are preferably applied to the hair directly after or just after the washing thereof. Between the washing and the application of the product the hair may optionally be rinsed with a hair conditioner and/or the hair may be towel dried or completely dried. Preferably the composition is applied to towel-dried hair. Another possibility is to apply the product to the hair at any moment between two wash occasions.

Accordingly the present invention also relates to a method of treating hair, wherein recently washed and optionally dried hair is treated with a hair-treatment composition comprising an astringent material and a viscosity control material.

After the application of the product the hair is not rinsed with water, but the product is left on the hair until the hair is washed again. Generally the period between two washing sessions will be 0.5 to 10 days, more generally 1-5 days.

The amount of composition which is applied to the hair is preferably from 0.1 to 25 mls, more preferred 3-20 mls, most preferred 5-15 mls.

The invention will be further illustrated by means of the following examples.

Example I

The ability of a polymeric thickener to control the viscosity of sebum was tested with pulsed NMR as follows:

Sebum was extracted from the hair. Clean hair samples were pretreated with a 3 % Lubrizol (an ethylene-styrene random copolymer ex Lubrizol) in ether and subsequently dried to produce hair fibres coated with 5 % by wt of the Lubrizol. Sebum dissolved in absolute alcohol was then absorbed onto the coated fibres and the solvent evaporated.

NMR measurements were carried out on a Bruke 322S pulse nmr spectrometer operating at 60 MHz. Transverse relaxation times $T_2$ were determined directly from free Induction Decay following a 90 ° r.f.

4

pulse or from Carr-Purcell (90-180$°_n$) echos.

The sebum of polymer-coated hair resulted in a broad peak in the $T_2$ spectrum between about 0.8 and 100 ms, while samples of sebum without polymer provided a sharp peak between 100 and 120 ms. These results indicate that polymeric thickeners are capable of reducing the $T_2$ relaxation time of sebum. It is believed that a reduced $T_2$ is an indication of reduced mobility and hence viscosity control of the sebum.

<u>Example II</u>

The following composition was made by mixing the ingredients to water in the listed order:

| Ingredient (% wt ) | |
|---|---|
| Zinc sulphate | 0.2 |
| Triclosan[1] | 0.05 |
| hydroxypropyl cellulose ether[2] | 0.4 |
| Ethanol | 20 |
| surfactant[3] | 0.8 |
| water | balance |

[1] Irgasan DP 300 ex Ciba-Geigy
[2] Klucel M ex Aqualon/Hercules, MS=3, Mw is 850,000
[3] Cremophor RH40 ex BASF

The composition was tested as follows: Hair was washed with 6g/head of Timotei*shampoo followed by rinsing with water and repeating the washing. The washed hair was towel-dried and 5 mls of the above composition was applied to one half of the hair, while 5 mls of a placebo (water) was applied to the other half of the hair. The hair was rubbed for 20 seconds and left for 2 minutes, whereafter the hair was dried and styled.

The greasiness of the two hair halfs after a period as indicated in Table I was judged by expert hairdressers on a scale of 0-4, wherein

0 = not greasy
1 = slightly greasy
2 = moderately greasy
3 = very greasy
4 = extremely greasy

Three features of greasiness were scored: (a) general impression on greasiness, (b) greasiness at the hair-roots and (c) degree of spreading of the sebum over the hair fibres. On each assessment day the hair was first judged on greasiness, whereafter the hair was washed and treated with the post-wash composition as indicated above.

Table I indicates the average difference between the treated half of the hair and the comparative half of the hair, whereby a positive score indicates a reduced greasiness when using the composition of the invention. A positive score of 0.32 or more indicates a more than 98 % certainty.

* trade mark

The following results were obtained:

| Table I | grease reduction | | |
| day | a | b | c |
| 1 | 0* | 0* | 0* |
| 2 | 0.24 | 0.07 | 0.02 |
| 5 | 0.33 | 0.26 | 0.24 |
| 8 | 0.35 | 0.28 | 0.22 |
| 10 | 0.48 | 0.55 | 0.50 |
| 12 | 0.55 | 0.66 | 0.52 |

* hair was assessed after washing on day 1;
the result shows that freshly washed hair is perceived
as non greasy

These results clearly indicate that a reduction in hair greasiness can be obtained by using a hair-treatment composition comprising an astringent material in combination with a viscosity control material for the sebum. The effects on greasiness become more pronounced if the composition is repeatedly used.

**Claims**

1. A hair treatment composition comprising:
   (a) from 10-30% by weight of an organic astringent
   (b) from 0.01 to 10% by weight of an inorganic astringent; and
   (c) a viscosity control material for sebum selected from anti-microbial materials and polymeric thickening materials.

2. A hair treatment composition according to claim 1, wherein the inorganic astringent material comprises an astringent zinc salt.

3. A hair treatment composition according to claim 2, wherein the organic astringent material is ethanol and the inorganic astringent material is selected from zinc sulphate and zinc chloride.

4. A hair treatment composition according to claim 1, wherein the viscosity control material comprises an anti-microbial agent selected from the group consisting of halogenated salicylanilides, halogenated carbanilides, halogenated bis-phenols, alkylbenzacrylates, quaternary ammonium compounds, thiuram sulfides, dithiocarbamates, pyridinone-N-oxides, halogenated diphenyl ethers, halogenated anilides of thiophene carboxylic acids and chlorohexidines.

5. A hair treatment composition according to claim 1, wherein the viscosity control material comprises a polymeric thickener selected from the group consisting of proteinaceous thickeners, esters of fatty acids and polyethylene glycols, gums, $C_{1-14}$ alkyl-substituted cellulose ethers, higher fatty acid esters of amylose, polysaccharides, depolymerised cellulose, dextran and fatty acid esters of polyglycerols.

6. A hair treatment composition according to claim 5, wherein the polymeric thickener is soluble in sebum.

7. Use of a composition according to any preceding claim for the treatment of hair to reduce greasiness thereof.

EP 0 490 583 B1

8. A method of treating hair to reduce greasiness thereof, wherein recently washed and optionally dried hair is treated with a hair treatment composition according to any preceding claim.

**Patentansprüche**

1. Haarbehandlungszusammensetzung mit
   (a) 10-30 Gew.-% eines organischen adstringierenden Mittels,
   (b) 0,01 bis 10 Gew.-% eines anorganischen adstringierenden Mittels und
   (c) einem aus antimikrobiellen Materialien und polymeren Dickungsmittelmaterialien ausgewählten, die Viskosität steuernden Material für Talg.

2. Haarbehandlungszusammensetzung nach Anspruch 1, wobei das anorganische adstringierende Material ein adstringierendes Zinksalz umfaßt.

3. Haarbehandlungszusammensetzung nach Anspruch 2, wobei das organische adstringierende Material aus Ethanol besteht und das anorganische adstringierende Material aus Zinksulfat und Zinkchlorid ausgewählt ist.

4. Haarbehandlungszusammensetzung nach Anspruch 1, wobei das die Viskosität steuernde Material ein antimikrobielles Material, ausgewählt aus der Gruppe halogenierte Salicylanilide, halogenierte Carbanilide, halogenierte Bisphenole, Alkylbenzacrylate, quaternäre Ammoniumverbindungen, Thiuramsulfide, Dithiocarbamate, Pyridinon-N-oxide, halogenierte Diphenylether, halogenierte Anilide von Thiophencarbonsäuren und Chlorohexidine, umfaßt.

5. Haarbehandlungszusammensetzung nach Anspruch 1, wobei das die Viskosität steuernde Material ein polymeres Dickungsmittel, ausgewählt aus der Gruppe proteinhaltige Dickungsmittel, Ester von Fettsäuren und Polyethylenglykolen, Gummis, ($C_1$-$C_{14}$)Alkyl-substituierte Celluloseether, höhere Fettsäureester von Amylose, Polysaccharide, depolymerisierte Cellulose, Dextran und Fettsäureester von Polyglyzerinen, umfaßt.

6. Haarbehandlungszusammensetzung nach Anspruch 5, wobei das polymere Dickungsmittel in Talg löslich ist.

7. Verwendung einer Zusammensetzung nach einem der vorhergehenden Ansprüche zur Behandlung von Haar zur Verringerung der Fettigkeit desselben.

8. Verfahren zur Behandlung von Haar zur Verringerung der Fettigkeit desselben, wobei frisch gewaschenes und gegebenenfalls getrocknetes Haar mit einer Haarbehandlungszusammensetzung nach einem der vorhergehenden Ansprüche behandelt wird.

**Revendications**

1. Composition de traitement de la chevelure comprenant:
   (a) de 10 à 30% en poids d'un astringent organique;
   (b) de 0,01 à 10% en poids d'un astringent minéral; et
   (c) une matière régulant la viscosité pour le sébum choisie parmi des matières anti-microbiennes et des matières épaississantes polymères.

2. Composition de traitement de la chevelure selon la revendication 1, dans laquelle la matière astringente minérale comprend un sel de zinc astringent.

3. Composition de traitement de la chevelure selon la revendication 2, dans laquelle la matière astringente organique est l'éthanol et la matière astringente minérale est choisie parmi le sulfate de zinc et le chlorure de zinc.

4. Composition de traitement de la chevelure selon la revendication 1, dans laquelle la matière régulant la viscosité comprend un agent anti-microbien choisi dans le groupe formé par les salicylanilides halogénés, les carbanilides halogénés, les bis-phénols halogénés, les alkylbenzoacrylates, les compo-

7

EP 0 490 583 B1

sés ammonium quaternaire, les sulfures de thiurame, les dithiocarbamates, les oxydes de N-pyridinone, les éthers diphényliques halogénés, les anilides halogénés d'acides thiophène carboxyliques et les chlorohexidines.

5. Composition de traitement de la chevelure selon la revendication 1, dans laquelle la matière régulant la viscosité comprend un épaississant polymère choisi dans le groupe formé par les épaississants protéiniques, les esters d'acides gras et de polyéthylèneglycols, les gommes, les éthers cellulosiques substitués par un groupe alkyle en $C_1$ à $C_{14}$, les esters d'acide gras supérieurs d'amylose, les polysaccharides, la cellulose dépolymérisée, le dextrane et les esters d'acide gras de polyglycérols.

6. Composition de traitement de la chevelure selon la revendication 5, dans laquelle l'épaississant polymère est soluble dans le sébum.

7. Utilisation d'une composition selon l'une quelconque des revendications précédentes pour le traitement des cheveux en vue de réduire leur capacité à être gras.

8. Procédé pour traiter les cheveux en vue de réduire leur capacité à être gras, dans lequel des cheveux récemment lavés et éventuellement séchés sont traités avec une composition de traitement de la chevelure selon l'une quelconque des revendications précédentes.

8